(19) Europäisches Patentamt
European Patent Office
Office européen des brevets

(11) EP 0 646 006 B1

(12) **FASCICULE DE BREVET EUROPEEN**

(45) Date de publication et mention de la délivrance du brevet:
**12.03.1997 Bulletin 1997/11**

(21) Numéro de dépôt: **93913137.1**

(22) Date de dépôt: **16.06.1993**

(51) Int. Cl.$^6$: **A61K 31/44**, A61K 9/50

(86) Numéro de dépôt international:
**PCT/FR93/00582**

(87) Numéro de publication internationale:
**WO 93/25204 (23.12.1993 Gazette 1993/30)**

# (54) COMPOSITIONS STABLES DE MICROGRANULES D'OMEPRAZOLE GASTRO-PROTEGES ET LEUR PROCEDE D'OBTENTION

IM MAGEN STABILE ZUSAMMENSETZUNGEN AUS OMEPRAZOLE MIKROGRANULATEN UND VERFAHREN ZU IHRER HERSTELLUNG

GASTRO-PROTECTED STABLE OMEPRAZOLE COMPOSITIONS IN THE FORM OF MICROGRANULES AND PROCESS FOR THEIR PRODUCTION

(84) Etats contractants désignés:
**AT BE CH DE DK FR GB GR IE IT LI LU MC NL PT SE**

(30) Priorité: **16.06.1992 FR 9207249**

(43) Date de publication de la demande:
**05.04.1995 Bulletin 1995/14**

(73) Titulaire: **ETHYPHARM**
**E-24004 Madrid (ES)**

(72) Inventeurs:
- **DEBREGEAS, Patrice**
  **F-75007 Paris (FR)**
- **LEDUC, Gérard**
  **F-45330 Malesherbes (FR)**

(74) Mandataire: **Ahner, Francis et al**
**CABINET REGIMBEAU**
**26, avenue Kléber**
**75116 Paris (FR)**

(56) Documents cités:
**EP-A- 0 237 506**
**EP-A- 0 324 981**
**EP-A- 0 470 047**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 646 006 B1

Printed by Rank Xerox (UK) Business Services
2.13.16/3.4

## Description

La présente invention concerne une formulation galénique d'oméprazole sous forme de microgranules gastro-protégés ayant une stabilité dans le temps compatible avec les exigences pharmaceutiques ainsi que le procédé de fabrication desdites microgranules.

L'oméprazole ou 5-méthoxy-2-[[(4-méthoxy-3,5-diméthyl-2-pyridinyl)méthyl]sulfinyl]-1H-benzimidazole, substance anti-ulcéreuse diminuant la sécrétion acide gastro-intestinale, est bien connue et a été notamment décrite dans le brevet suédois n° 78 04231 (Aktiebolaget Hässle Fack).

Il est également connu que l'oméprazole possède une solubilité très faible dans l'eau, mais qu'il est par contre très soluble dans les solutions alcalines et qu'il se dégrade très vite en milieu acide. Sa demi-vie de dégradation est de dix minutes dans des solutions aqueuses de pH inférieur à 4, de dix-huit heures pour un pH égal à 6,5 et d'environ 300 jours pour un pH de 11.

Lorsque l'on utilise l'oméprazole par voie orale, il est par conséquent indispensable de gastro-protéger la forme galénique de la matière active (gélules, comprimés ou poudre) de façon à éviter tout contact de celle-ci en milieu acide au niveau gastrique tout en réalisant une dissolution rapide en milieu intestinal, niveau auquel l'oméprazole doit être absorbé, c'est-à-dire, dès que le pH ambiant devient supérieur à 6,8.

Les caractéristiques de dissolution in vitro d'une formulation orale d'oméprazole ainsi que les résultats de biodisponibilité réalisés chez l'homme à l'aide de ces formulations orales, constitués de granules protégés ont été décrites dans le Journal de Gastro-entérologie Scandinave (1985 ; 20 (suppl. 108) : 113-120, Pilbran A. Cederberg C).

Toutefois, il a été constaté que les granules entériques classiques ne présentaient pas une stabilité dans le temps suffisante pour être utilisés dans une forme pharmaceutique satisfaisante (gélules ou comprimés de microgranules), puisqu'on observe rapidement une dégradation du principe actif lorsqu'ils sont placés dans des conditions d'utilisation habituelles (température ambiante de 25°C, degré d'humidité compris entre 40 et 75%), avec l'apparition de produits de dégradation nocifs, tels que décrits par exemple dans Acta Chemica Scandinavia 43 (1989) 536-548 (Arne BRÄNDSTROM & AL.).

Pour pallier ces inconvénients, il a notamment été proposé (EP-A-0 247 983 HÄSSLE A-G), de mélanger dans une masse de dérivés cellulosiques d'enrobage et de désintégrants une quantité adéquate d'oméprazole et d'un sel alcalin de sodium, potassium, magnésium, calcium ou les sels d'ammonium alcalins, de façon à obtenir par extrusion un noyau dont la composition est tamponnée et possède un pH alcalin compris entre 7 et 12.

Les microgranules ainsi réalisés sont alors gastro-protégés classiquement et répondent aux normes de dissolution et de gastro-résistance définies pour une utilisation optimale du produit par voie orale, c'est-à-dire une dissolution supérieure à 75% après 30 minutes dans un milieu aqueux de pH égal à 6,8 et une gastro-résistance supérieure à 85% après 2 heures dans un milieu de pH égal à 1,2.

Néanmoins, ce type de réalisation présente plusieurs inconvénients importants, tant du point de vue technique qu'économique. Tout d'abord, cela oblige à préparer des associations de produits et des mélanges, avec des risques de toxicité globale et d'instabilité au cours du procédé de fabrication. En outre, il faut utiliser un type d'appareils spécifiques coûteux ajoutant au coût élevé du principe actif, ce qui tend à augmenter par trop le prix du produit fini pour une utilisation massive au niveau mondial, le coût de traitement journalier lié à l'utilisation d'un tel produit étant beaucoup plus important que celui obtenu en utilisant un traitement du type sel de bismuth et métronidazole ou macrolide agissant comme anti-acide et agents destructeurs de l'Hélicobacter pylori (agent responsable de la majorité des ulcérations gastro-intestinales).

L'objet de l'invention remédie à ces inconvénients. Il concerne une nouvelle formulation stable de microgranules gastro-protégés d'oméprazole et le procédé permettant de les obtenir, dans des conditions économiques avantageuses.

Selon l'invention, l'oméprazole est utilisé sous forme de poudre diluée dans une quantité sensiblement égale de mannitol de façon à obtenir une suspension homogène et stable et de façon à assurer une meilleure uniformité de la teneur en oméprazole lors de l'application sur des grains neutres constitués d'un mélange de sucre et d'amidon.

La présente invention concerne donc une formulation stable de microgranules d'oméprazole caractérisés en ce qu'ils comportent un noyau neutre constitué de sucre et d'amidon enrobé d'une couche active comprenant une dilution d'oméprazole dans du mannitol en quantités sensiblement égales.

Par poudre diluée, on entend selon l'invention un mélange d'une poudre d'oméprazole, d'une poudre de mannitol, de lauryl sulfate de sodium et de carboxy méthylamidon.

D'une manière préférentielle, les poudres mélangées ont une granulométrie sensiblement égale, inférieure à 100 µm.

Par quantités sensiblement égales, on entend un rapport en poids poudre d'oméprazole/ poudre de mannitol voisin de 1, c'est-à-dire compris entre 0,9 et 1,2.

Les noyaux neutres constitués de sucre et d'amidon dans des proportions en poids voisines de 4 parties pour 1 partie d'oméprazole poudre, ont un diamètre moyen compris entre 0,7 mm et 0,9 mm.

La couche de principe actif, ou couche active appliquée sur les grains neutres aura de préférence une épaisseur

comprise entre 100 et 300 μm environ (0,1 et 0,3 mm).

Pour assurer la bonne stabilité du principe actif au cours du stockage des microgranules selon l'invention, le taux d'humidité de la couche active est inférieur à 1 %, de préférence inférieur à 0,5%.

En outre, le microgranule selon l'invention comprend avantageusement une couche de protection complémentaire de la couche active constituée de mannitol et d'un agent liant, afin d'isoler définitivement le noyau sur lequel est appliqué l'oméprazole de la couche externe d'enrobage destinée à assurer la gastroprotection des noyaux actifs.

D'une manière préférentielle, l'agent liant est de l'hydroxypropylméthylcellulose (HPMC), alors que la couche gastro-résistante comprend du phtalate d'hydroxypropylméthyl cellulose (HPMCP) ou des copolymères ammoniques de l'acide méthacrylique et du talc.

Pour satisfaire aux critères de dissolution in vitro des microgranules d'oméprazole selon lesquels au moins 75% de l'oméprazole doit être dissous au bout de 30 minutes lorsque l'on place les microgranules dans un milieu aqueux de pH égal à 6,8 et à une température de 37°C, on ajoute dans le mélange initial d'oméprazole et de mannitol environ 10% de carboxy-méthyl-amidon (EXPLOTAB® commercialisé par la société MENDELL) afin d'obtenir une désagrégation complète des granules à pH égal à 6,8, ainsi qu'environ 5% d'un agent tensio-actif tel que le lauryl sulfate de sodium qui améliore la solubilisation de l'oméprazole dans le milieu intestinal.

De plus, on utilise comme solutions liantes pour appliquer la couche active sur les grains neutres puis la protection complémentaire de mannitol, des solutions à viscosité forte de l'ordre de 15 centipoises ($15.10^{-3}$ P.s.) d'hydroxypropylméthylcellulose (HPMC) (PHARMACOAT® commercialisé par la société SHINETSU) utilisées en solution dans un mélange d'au moins 80% d'éthanol et au plus 20% d'eau, par exemple un mélange de 10 parties d'eau pour 90 parties d'éthanol à 95° et selon des faibles quantités afin de limiter au maximum la teneur en solvant et en eau des granules tout au long de la fabrication des granules actifs.

Enfin, selon un mode de réalisation des granules stables d'oméprazole selon l'invention, l'enrobage gastro-résistant des microgranules constitué de phtalate d'hydroxypropylméthylcellulose (HPMCP) (HP 50 R commercialisé par la Société SHIN-ETSU) en solution à 7,5 % dans un mélange 80-20 en masse d'acétone et d'éthanol à 95°, est appliqué en couches externes après un prémontage des microgranules actifs d'oméprazole et de mannitol par pulvérisation d'un sirop de saccharose à 33%, dans un mélange eau/éthanol de 44/56 en masse, ce sirop de sucre ayant un poids total en saccharose d'environ 25% par rapport à la quantité d'enrobage final gastrorésistant. Cette couche extérieure peut comporter du talc comme lubrifiant.

Pendant toute la durée des différentes phases d'application et d'enrobage, la température des granules est maintenue entre 32°C et 38°C et entre chaque phase, on réalise un séchage à une température comprise entre 35°C et 40°C de façon à ce que les teneurs en solvants et en eau soient minimales, la teneur finale étant inférieure à 1% pour l'eau et 2000 ppm pour l'éthanol.

D'autres caractéristiques et avantages de l'invention apparaîtront à la lumière des exemples ci-après.

<u>Exemple 1</u>: Procédé de préparation de Microgranules d'Oméprazole dosés à 8,41 % en poids.

Toutes les quantités mentionnées sont exprimées en kg de masse sèche pour une quantité finale égale à 237,80 kg de microgranules ayant une teneur de 84,1 mg d'oméprazole par gramme de microgranules.

COMPOSITION

| | |
|---|---|
| Grains neutres de sucre et d'amidon | 80,00 |
| Couche active: | |
| Oméprazole | 20,00 |
| Mannitol | 20,00 |
| Carboxyméthyl amidon (CMA) | 4,00 |
| Lauryl sulfate de sodium | 2,00 |
| Couche de protection complémentaire | |
| Mannitol | 50,00 |
| Saccharose | 8,00 |
| HPMC 15 centipoises | 3,20 |
| Couche gastro-résistante | |
| HPMCP | 32,00 |
| Talc | 18,60 |
| | 237,80 |

Le procédé, selon l'invention, consiste à préparer une dilution de 20 kg d'oméprazole dans 20 kg de mannitol en présence de 4 kg de CMA et de 2 kg de lauryl sulfate de sodium, puis à lier cette dilution sèche sur 80 kg de grains neutres de sucre et d'amidon dont le diamètre est compris entre 0,7 et 0,9 mm à l'aide d'une solution de HPMC (1,6kg) à 4% dans l'eau (20 parties) et l'éthanol à 95 ° (80 parties) dans une turbine circulaire à fond plat incliné, puis à protéger les grains actifs ainsi obtenus à l'aide de 50 kg de mannitol appliqués avec le reste de la solution de HPMC (soit 1,6 kg), puis à prémonter les granules ainsi protégés par un sirop de sucre (8 kg de saccharose) à 33% constitué de 44 parties d'eau pour 56 parties d'éthanol à 95° et à appliquer enfin l'enrobage de gastroprotection à raison de 32 kg de HPMCP en solution à 7,5% dans un mélange de 80 parties d'éthanol à 95° pour 20 parties d'acétone.

Les granules ainsi obtenus montrent une gastrorésistance supérieure à 85% dans un milieu de pH 1,2, puisqu'au bout de 2 heures à 37°C, la quantité d'oméprazole non libérée dans ce milieu est de 91,7%. Dans un milieu aqueux de pH égal à 6,8, la dissolution des granules montre au bout de 30 minutes un taux d'oméprazole de 92%, soit une quantité supérieure à 75% qui est la norme minimale acceptable.

Au bout de 12 et 24 mois, les valeurs respectives de gastrorésistance sont de 92,1% et 92,8% et les dissolutions à pH 6,8 de 91,4% et 93,8% dans les mêmes conditions opératoires.

De plus, la couleur beige très claire des microgranules conservés en gélules à température ambiante et une humidité extérieure de 60% dans des flacons de verte à opercule munis de capsule déshydratante, est maintenue constante pendant une durée d'au moins 24 mois.

Exemple 2: Tests de Stabilité

L'étude de la stabilité de l'Oméprazole a été réalisée sur 4 lots de gélules comprenant les microgranules selon l'invention et un lot de microgranules. Les lots contrôlés, les conditions de conservation, les méthodes d'analyses ainsi que les résultats sont présentés ci-après.

1. LOTS CONTROLES

Oméprazole gélules

C458-1-2, C458-1-6, C458-2-3 et C458-2-4

Oméprazole microgranules

UQM 001-3

| LOTS | DATES DE FABRICATION | TEMPS DE CONSERVATION (mois) |
|---|---|---|
| C458-1-2 | Juin 1983 | 36 |
| C458-1-6 | Février 1984 | 36 |
| C458-2-3 | Septembre 1984 | 24 |
| C458-2-4 | Octobre 1984 | 24 |
| UQM 001-3 | Octobre 1990 | 3 |

2. CONDITIONS DE CONSERVATION

- 25°C - Humidité relative 60%
- 30°C - Humidité relative 30 - 40%, 60%
- 37°C - Humidité relative 20 - 30%, 90%
- 50°C - Humidité relative 80%
- Réfrigérateur

3. ANALYSE DES ECHANTILLONS

a. gélules

Solution d'échantillon

A - Pour le dosage de l'oméprazole, introduire le contenu de 5 gélules dans une fiole jaugée de 250 ml, puis introduire 200 ml de solution de méthanol-ammoniaque (95/5), et agiter magnétiquement durant 30 minutes. Compléter à l'aide du même solvant. Filtrer et diluer 10 ml de filtrat dans une fiole jaugée de 100 ml, avec 30 ml de dichlorométhane et compléter au volume à l'aide d'une solution méthanol-ammoniaque-dichlorométhane (24/1/75).

B - Pour la détermination des produits de dégradation, dissoudre le contenu de 2 gélules dans 8 ml de la solution de méthanol-ammoniaque (95/5), sous agitation magnétique pendant 30 minutes. Filtrer et diluer 2,5 ml du filtrat à 100 ml dans du dichlorométhane. Injecter immédiatement.

Appareillage et conditions

- Appareil CLHP équipé d'un détecteur U.V.
- Précolonne de silice, 7µm, de 15 mm de longueur x 3,2 mm de diamètre interne (Brownlee ou équivalent).
- Colonne de Lichrosorb Si 60, 5µm, de 124 mm de longueur x 4 mm de diamètre interne (Hibar Merck ou équivalent).
- Débit : 1 ml/min.
- Lecture à 280 nm
- injection : 40 µl.

Contrôle du système

Une fois la stabilité de la ligne de base obtenue, le système est contrôlé de la manière suivante :

Dissoudre 5 mg d'oméprazole standard et 5 mg de la sulfone correspondante H 168/66 (dont la formule développée est reproduite ci-après) dans 100 ml de phase mobile. Injecter plusieurs fois 40 µl jusqu'à l'obtention d'une constante des temps de rétention (différence entre deux injections <1%).

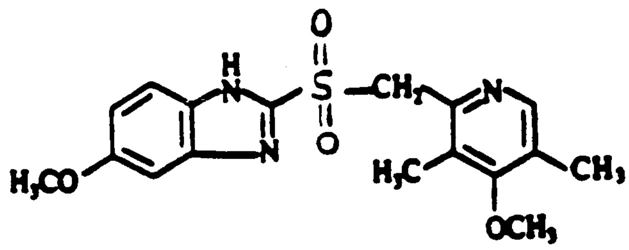

Le temps de rétention de l'oméprazole est de 10 minutes et celui de la sulfone H 168/66 de 8 minutes.

b. microgranules

Détermination de la teneur en oméprazole des microgranules

La teneur en oméprazole des microgranules est déterminée par spectrophotométrie dans l'ultra-violet après séparation par chromatographie liquide haute peformance.

Réactifs

- Acétonitrile pour CLHP (par exemple CARLO ERBA réf. 412409)
- phosphate de sodium dibasique ($Na_2HPO_4$)
- Phosphate de sodium monobasique ($NaH_2PO_4$, $H_2O$)
- Eau distillée
- Substance de référence : OMÉPRAZOLE

Appareillage

Appareil pour chromatographie liquide avec détecteur UV (280 nm) :

* 2 colonnes en série, en acier inoxydable :

  - précolonne : longueur 1,5 cm - ∅ interne 3,2 mm - granulométrie 7µm
  - colonne : longueur 12,5 cm - ∅ interne 4 mm - granulométire 5 µm

* Phase stationnaire : Lichrosorb RP 18
* Système d'injection : pour des quantités de 20 µl (par exemple injecteur automatique WISP 712).
* Intégrateur : LCI 100 PERKIN ELMER ou WATERS 645 DATA MODULE

Essais de gastro-résistance et de dissolution

Les microgranules sont soumis, pendant 2h30, à une agitation dans des milieux appropriés à température constante (37°C +/- 0,5°C) dans un appareil de dissolution.

Appareillage

L'appareil de dissolution utilisé est l'appareil à palette décrit à la Pharmacopie Européenne.

| | |
|---|---|
| Vitesse de rotation : | 100 tours / minute |
| Volume utilisé : | 500 ml de milieu de pH 1,2. |
| | Ajout de 400 ml de milieu de pH 7,6. |

Milieux

Milieu I : Milieu de résistance acide.

Introduire dans une fiole jaugée de 1 litre, 2 grammes de chlorure de sodium et 7 ml d'acide chlorhydrique concentré (37%). Compléter au volume à l'aide d'eau purifiée. Agiter jusqu'à dissolution. Le pH de cette solution doit être 1,2 +/- 0,05.
Milieu II : Milieu de pH 7,6 (ajout)
Solution de phosphate sodique dibasique ($Na_2HPO_4$) 0,235 M.
Préparer 1 litre de solution pour 2 vases.

4. RESULTATS ET CONCLUSIONS

Les résultats sont résumés dans les tableaux ci-après.

Lors de la détermination de la teneur en impuretés, deux impuretés principales sont détectées sur tous les lots. Dans les tableaux de résultats, il sont identifiées sous les dénominations I et I'.

A : Période de conservation (mois)
B : Conditions de conservation - ° C Humidité relative
C : Aspect

| Oméprazole gélules lot C458-1-2 | | | | | | | | |
|---|---|---|---|---|---|---|---|---|
| **A** | **B** | | **C** | **D** | **E** | **F** | | |
| | | | | | | *I %* | *I' %* | *Autres %* |
| 0 | | | a | 6 | 20,3 | <0,5 | <0,5 | <0,2 |
| 6 | 25 | 60 | a | 5 | 20,6 | <0,5 | <0,5 | <0,2 |
| | 30 | 60 | a | 7 | 20,1 | <0,5 | <0,5 | <0,2 |
| | 50 | 80 | c | 7 | 19,4 | <0,5 | <0,5 | <0,3 |
| 12 | 25 | 60 | a | 5 | 20,5 | <0,5 | <0,5 | <0,2 |
| | 30 | 60 | b | 6 | 20,5 | <0,5 | <0,5 | <0,2 |
| 18 | 25 | 60 | a | 6 | 19,8 | <0,5 | <0,5 | <0,2 |
| | 30 | 60 | b | 6 | 20,1 | <0,5 | <0,5 | <0,2 |
| 24 | 25 | 60 | a | 6 | 20,7 | <0,5 | <0,5 | <0,2 |
| | 30 | 60 | c | 7 | 20,2 | <0,5 | <0,5 | <0,3 |
| 30 | 25 | 60 | a | 6 | 20,3 | <0,5 | <0,5 | <0,3 |
| 36 | 25 | 60 | b | 6 | 19,9 | <0,5 | <0,5 | <0,3 |
| | réfrigérateur | | a | 6 | 20,8 | <0,5 | <0,5 | <0,2 |
| a = blanc, b = coloré, conforme, c = coloré, non conforme | | | | | | | | |

| Oméprazole gélules lot C458-1-6 | | | | | | | | |
|---|---|---|---|---|---|---|---|---|
| A | B | | C | D | E | F | | |
| | | | | | | I % | I' % | Autres % |
| 0 | | | a | 5 | 19,4 | <0,5 | <0,5 | <0,2 |
| 6 | 25 | 60 | a | 5 | 19,6 | <0,5 | <0,5 | <0,2 |
| | 30 | 60 | a | 6 | 19,5 | <0,5 | <0,5 | <0,2 |
| | 50 | 80 | c | 7 | 18,7 | <0,5 | <0,5 | <0,4 |
| 12 | 25 | 60 | a | 5 | 19,2 | <0,5 | <0,5 | <0,2 |
| | 30 | 60 | b | 6 | 19,3 | <0,5 | <0,5 | <0,2 |
| 18 | 25 | 60 | a | 5 | 19,7 | <0,5 | <0,5 | <0,2 |
| | 30 | 60 | b | 5 | 19,1 | <0,5 | <0,5 | <0,3 |
| 24 | 25 | 60 | a | 6 | 19,0 | <0,5 | <0,5 | <0,2 |
| | 30 | 60 | c | 6 | 19,2 | <0,5 | <0,5 | <0,3 |
| 30 | réfrigérateur | | a | 6 | 19,8 | <0,5 | <0,5 | <0,2 |
| | 25 | 60 | a | 5 | 19,3 | <0,5 | <0,5 | <0,2 |
| 36 | réfrigérateur | | a | 6 | 19,8 | <0,5 | <0,5 | <0,2 |
| | 25 | 60 | b | 5 | 19,2 | <0,5 | <0,5 | <0,2 |
| a = blanc, b = coloré, conforme, c = coloré, non conforme | | | | | | | | |

| Oméprazole gélules lot C458-2-3 | | | | | | | | |
|---|---|---|---|---|---|---|---|---|
| A | B | | C | D | E | F | | |
| | | | | | | I % | I' % | Autres % |
| 0 | | | a | <5 | 21,2 | <0,5 | <0,5 | <0,2 |
| 6 | réfrigérateur | | a | <5 | 20,9 | <0,5 | <0,5 | <0,2 |
| | 25 | 60 | a | 5 | 21,1 | <0,5 | <0,5 | <0,2 |
| 12 | réfrigérateur | | a | 5 | 20,8 | <0,5 | <0,5 | <0,2 |
| | 25 | 60 | a | <5 | 20,7 | <0,5 | <0,5 | <0,2 |
| 18 | réfrigérateur | | a | <5 | 20,6 | <0,5 | <0,5 | <0,2 |
| | 25 | 60 | a | 5 | 20,4 | <0,5 | <0,5 | <0,2 |
| 24 | 25 | 60 | a | 6 | 20,7 | <0,5 | <0,5 | <0,2 |
| a = blanc, b = coloré, conforme, c = coloré, non conforme | | | | | | | | |

| Oméprazole gélules lot C458-2-4 | | | | | | | | |
|---|---|---|---|---|---|---|---|---|
| A | B | | C | D | E | F | | |
| | | | | | | *I %* | *I' %* | *Autres %* |
| 0 | | | a | 6 | 20,7 | <0,5 | <0,5 | <0,2 |
| 6 | 25 | 60 | a | <5 | 20,8 | <0,5 | <0,5 | <0,2 |
| 12 | 25 | 60 | a | 5 | 20,3 | <0,5 | <0,5 | <0,2 |
| 18 | 25 | 60 | a | 6 | 20,5 | <0,5 | <0,5 | <0,2 |
| 24 | réfrigérateur | | a | 5 | 20,0 | <0,5 | <0,5 | <0,2 |
| | 25 | 60 | b | 5 | 20,4 | <0,5 | <0,5 | <0,5 |
| a = blanc, b = coloré, conforme, c = coloré, non conforme | | | | | | | | |

## Oméprazole microgranules UQM 001-3

A : Période de conservation (mois)
B : Conditions de conservation - ° C Humidité relative
C : Aspect
D : Dosage de l'oméprazole (mg/g)
E : Essai de gastrorésistance
F : Essai de Dissolution - %

| A | B | | C | D | E | F |
|---|---|---|---|---|---|---|
| 0 | | | a | 78,0 | 95,9 | 95,0 |
| 1 | 25 | 55-65 | a | 80,8 | 93,9 | 94,0 |
| | 30 | 30-40 | a | 81,2 | 94,4 | 95,0 |
| | 37 | 20-40 | a | 80,4 | 97,0 | 97,0 |
| | 37 | 90 | a | 77,6 | 97,5 | 100,0 |
| 3 | 25 | 56-65 | a | 81,4 | 91,6 | 92,0 |
| | 30 | 30-40 | a | 79,1 | 95,7 | 96,0 |
| | 37 | 20-40 | a | 80,2 | 92,0 | 93,0 |
| | 37 | 90 | c | 41,9 | - | - |

a = blanc, b = coloré, conforme, c = coloré, non conforme

Les gélules ont une bonne stabilité au réfrigérateur et à 25°C, jusqu'à 36 mois de conservation. Toutes les impuretés ont une concentration inférieure à 0,50%.

La teneur en impuretés augmente un peu plus avec la température et l'humidité, et peut être suffisante pour provoquer une coloration des granules.

Les microgranules conservés en vrac ont une bonne stabilité pendant 3 mois à température ambiante et humidité relative 55-65%, à 30°C et humidité relative 30-40% et à 37°C et humidité relative 20-30%, et pendant 1 mois à 37°C et humidité relative 90%.

DUREE DE VALIDITE

A la vue des résultats obtenus, la spécialité est stable pendant 36 mois, à condition de ne pas dépasser 25°C durant cette période.

NORMES

| | |
|---|---|
| 1. Aspect | gélules de gélatine dure contenant des granules blanchâtres à beige plus ou moins foncé sans particules étrangers. |
| 2. Poids moyen | environ 235 mg ≠ 10% |
| 3. Teneur en oméprazole | 18,0 - 22,0 mg/gélule (90 - 110%) |
| 4. Résistance en milieu acide | ≥ 85 % |
| 5. Libération à pH 6,8 | ≥75 % |
| 6. Produits de dégradation | teneur totale ≤ 2,0% |
| | teneurs individuelles ≤ 0,5% |

Bien entendu, l'homme de l'art pourra apporter des variantes dans les modes de réalisation des microgranules selon l'invention, en particulier, en utilisant des appareils d'enrobage tels que des lits fluidisés à la place des turbines à fonds plats décrites ci-dessus, ou encore en utilisant des polymères méthacryliques comme matériaux de gastroprotection (par exemple l'EUDRAGIT® L 100-55 ou L 30 D de la société ROHM & HASS) ou d'autres polymères gastro-résistants sans pour cela sortir du cadre et de la portée de l'invention.

Par contre, l'invention n'est pas destinée à réaliser des microgranules par les techniques d'extrusion classiques déjà décrites dans l'art antérieur, ces techniques nécessitant d'utiliser l'oméprazole en solution dans une masse contenant des solvants et de l'eau, ce qu'on évite grâce aux modes de réalisation du procédé décrit ci-dessus afin de pallier aux conséquences de l'instabilité de l'oméprazole dans de telles conditions.

**Revendications**

**1.** Formulation stable de microgranules d'oméprazole comportant un noyau neutre constitué de sucre et d'amidon, caractérisée en ce qu'elle comporte une couche active constituée d'une dilution d'oméprazole dans du mannitol dans un rapport en poids oméprazole/mannitol voisin de 1, et une couche externe de gastro-protection constituée d'un enrobage gastro-résistant du type phtalate d'hydroxypropylméthylcellulose et de talc.

**2.** Formulation selon la revendication 1, caractérisée en ce que la couche active d'oméprazole comporte sensiblement 10% en poids de carboxyméthylamidon.

**3.** Formulation selon la revendication 2, caractérisée en ce que la couche active d'oméprazole comporte sensiblement 5% d'un composé tensio-actif tel que le lauryl sulfate de sodium.

**4.** Formulation selon l'une quelconque des revendications 1 à 3, caractérisée en ce qu'elle comporte à la surface de la couche active d'oméprazole une couche complémentaire de protection constituée de mannitol.

**5.** Formulation selon l'une quelconque des revendications 1 à 4, caractérisée en ce que la dilution d'oméprazole dans du mannitol et ladite couche de protection sont appliquées au moyen d'un liant du type hydroxypropylméthylcellulose de forte viscosité.

**6.** Microgranules d'oméprazole comportant un noyau neutre constitué de sucre et d'amidon, caractérisés en ce qu'ils comportent une couche active constituée d'une dilution d'oméprazole dans du mannitol dans un rapport en poids oméprazole/mannitol voisin de 1.

**7.** Procédé d'obtention de formulation selon l'une quelconque des revendications 1 à 5, caractérisé en ce qu'on applique sur des grains neutres constitués de sucre et d'amidon une dilution sèche de mannitol et d'oméprazole à l'aide d'une solution liante de type hydroxypropylméthylcellulose de viscosité forte en solution dans un mélange d'au moins 80% d'éthanol et au plus 20% d'eau, puis en ce que l'on applique la couche complémentaire de protection et/ou l'enrobage gastro-résistant.

**8.** Procédé selon la revendication 7, caractérisé en ce que chaque application de la dilution sèche est suivie d'un

séchage à une température comprise entre 35°C et 40°C pendant un temps permettant d'abaisser la teneur en eau des microgranules actifs à 1% et leur teneur en éthanol à 2000 ppm.

9. Procédé selon les revendications 7 et 8, caractérisé en ce qu'on utilise des microgranules neutres dont la taille est comprise entre 0,7 et 0,9 mm.

10. Procédé selon les revendications 7, 8 et 9, caractérisé en ce qu'on utilise pour réaliser les applications de dilution active et les enrobages de gastro-protection, des turbines à fond plat.

## Claims

1. Stable formulation of omeprazole microgranules containing a neutral core consisting of sugar and starch, characterized in that it contains an active layer composed of a dilution of omeprazole in mannitol in an omeprazole/mannitol ratio by weight in the region of 1 and an external gastroprotection layer composed of a gastroresistant coating of hydroxypropyl methylcellulose phthalate type and of talc.

2. Formulation according to Claim 1, characterized in that the active omeprazole layer contains substantially 10 % by weight of carboxymethylstarch.

3. Formulation according to Claim 2, characterized in that the active omeprazole layer contains substantially 5 % of a surface-active compound such as sodium lauryl sulphate.

4. Forumlation according to any one of Claims 1 to 3, characterized in that it contains, at the surface of the active omeprazole layer, an additional protective layer composed of mannitol.

5. Formulation according to any one of Claims 1 to 4, characterized in that the dilution of omeprazole in mannitol and the said protective layer are applied by means of a high-viscosity binder of hydroxypropyl methylcellulose type.

6. Omeprazole microgranules containing a neutral core consisting of sugar and starch, characterized in that they contain an active layer composed of a dilution of omeprazole in mannitol in an omeprazole/mannitol ratio by weight in the region of 1.

7. Process for producing a formulation according to any one of Claims 1 to 5, characterized in that a dry dilution of mannitol and of omeprazole is applied to neutral grains consisting of sugar and of starch with the aid of a high-viscosity binding solution of hydroxypropyl methylcellulose type in solution in a mixture of at least 80 % ethanol and at most 20 % water and in that the additional protective layer and/or the gastroresistant coating is/are then applied.

8. Process according to Claim 7, characterized in that each application of the dry dilution is followed by drying at a temperature between 35°C and 40°C for a time which makes it possible to lower the water content of the active microgranules to 1 % and their ethanol content to 2000 ppm.

9. Process according to Claims 7 and 8, characterized in that neutral microgranules are used whose size is between 0.7 and 0.9 mm.

10. Process according to Claims 7, 8 and 9, characterized in that flat-bottomed turbines are used to carry out the active dilution applications and the gastroprotection coatings.

## Patentansprüche

1. Stabile Formulierung aus Omeprazol-Mikrogranulaten, umfassend einen aus Stärke und Zucker bestehenden neutralen Kern, dadurch charakterisiert, daß sie eine aktive Schicht bestehend aus einer Omeprazolverdünnung in Mannitol in einem Gewichtsverhältnis Omeprazol/Mannitol nahe 1, und eine äußere Magensaft-Schutzschicht aus einer magensaftbeständigen Umhüllung vom Typ Hydroxypropylmethylcellulosephthalat und Talkum umfaßt.

2. Formulierung nach Anspruch 1, dadurch charakterisiert, daß die aktive Omeprazol-Schicht 10 Gew.-% Carboxymethylstärke umfaßt.

3. Formulierung anch Anspruch 2, dadurch charakterisiert, daß die aktive Omeprazol-Schicht 5 % einer oberflächenaktiven Verbindung wie Natriumlaurylsulfat umfaßt.

4. Formulierung nach einem der Ansprüche 1 bis 3, dadurch charakterisiert, daß sie an der Oberfläche der aktiven Omeprazol-Schicht eine zusätzliche aus Mannitol bestehende Schutzschicht umfaßt.

5. Formulierung nach einem der Ansprüche 1 bis 4, dadurch charakterisiert, daß die Verdünnung aus Omeprazol in Mannitol und die Schutzschicht mit Hilfe eines Bindemittels vom Typ Hydroxymethylcellulose mit großer Viskosität aufgebracht werden.

6. Omeprazol-Mikrogranulate, umfassend einen neutralen Kern aus Stärke und Zucker, dadurch charakterisiert, daß sie eine aktive Schicht aus einer Verdünnung aus Omeprazol in Mannitol in einem Gewichtsverhältnis Omeprazol/Mannitol nahe 1 umfassen.

7. Verfahren zur Herstellung der Formulierung nach einem der Ansprüche 1 bis 5, dadurch charakterisiert, daß man auf neutrale Körner, bestehend aus Stärke und Zucker, eine trockene Verdünnung von Mannitol und Omeprazol mit Hilfe einer bindenden Lösung vom Typ Hydroxymethylcellulose mit großer Viskosität, gelöst in einer Mischung aus mindestens 80 % Ethanol und höchstens 20 % Wasser aufträgt und anschließend die zusätzliche Schutzschicht und/oder die magensaftbeständige Umhüllung aufträgt.

8. Verfahren nach Anspruch 7, dadurch charakterisiert, daß jedes Auftagen der trockenen Verdünnung gefolgt wird von Trocknen bei einer Temperatur zwischen 35 und 40 °C während einer Zeit, die es ermöglicht, den Wassergehalt der aktiven Mikrogranulate auf 1 % und ihren Ethanolgehalt auf 2000 ppm zu senken.

9. Verfahren nach den Ansprüchen 7 und 8, dadurch charakterisiert, daß man neutrale Mikrogranulate verwendet, deren Größe zwischen 0,7 und 0,9 mm liegt.

10. Verfahren nach den Ansprüchen 7, 8 und 9, dadurch charakterisiert, daß man zum Auftragen der aktiven Verdünnung und der Magensaft-Schutzmhüllung Turbinen mit flachem Boden verwendet.